Europäisches Patentamt

European Patent Office

Office européen des brevets

(11) Publication number: **0 058 093**
**B1**

(12) **EUROPEAN PATENT SPECIFICATION**

(45) Date of publication of patent specification: **24.10.84**

(21) Application number: **82300661.4**

(22) Date of filing: **10.02.82**

(51) Int. Cl.³: **C 12 P 21/00,** A 61 K 37/02 //
C12R1/645

(54) Glycoprotein having anti-tumour and lectin-like activity.

(30) Priority: **10.02.81 JP 18655/81**

(43) Date of publication of application:
**18.08.82 Bulletin 82/33**

(45) Publication of the grant of the patent:
**24.10.84 Bulletin 84/43**

(84) Designated Contracting States:
**BE DE FR GB IT SE**

(56) References cited:
**GB-A-1 331 513**
**GB-A-1 521 168**
**GB-A-1 538 399**
**GB-A-1 565 090**
**GB-A-1 570 538**
**US-A-4 188 384**
**US-A-4 202 969**

(73) Proprietor: **KUREHA KAGAKU KOGYO
KABUSHIKI KAISHA
9-11 Horidome-cho 1-chome Nihonbashi Chuo-
ku
Tokyo (JP)**

(72) Inventor: **Yoshikumi, Chikao
2-19-46 Higashi
Kunitachi-shi Tokyo (JP)**
Inventor: **Fujii, Takayoshi
5-11-21 Towa
Adachi-ku Tokyo (JP)**
Inventor: **Furusho, Takao
1-6-13 Asahi-cho
Machida-shi Tokyo (JP)**
Inventor: **Matsunaga, Kenichi
3-26-1 Hyakunin-cho
Shinjuku-ku Tokyo (JP)**
Inventor: **Ohara, Minoru
19-25 Fujimi-cho
Itabashi-ku Tokyo (JP)**
Inventor: **Kobayashi, Akira
5-31-1 Matsubara
Setagaya-ku Tokyo (JP)**

(74) Representative: **Myerscough, Philip Boyd et al
J.A.Kemp & Co. 14, South Square Gray's Inn
London, WC1R 5EU (GB)**

Courier Press, Leamington Spa, England.

**Description**

The present invention relates to a glycoprotein having an anti-tumour activity and a lectin-like activity, to its production and to pharmaceutical compositions containing it.

The term "lectin-like activity" is used for describing generically cancer cell-specific cell-agglutinating activity and blast-transforming activity to lymphocytes, both of which are inherent in substances referred to as lectins, plus a non-specific inhibitory function on antigen-specific erythrocyte agglutination, affinity to the surface of lymphocytes and activity to macrophages.

Previously, the present inventors had prepared a substance having an antitumour activity by extracting a basidiomycetous fungus belonging to the genus *Coriolus* with hot water or an aqueous alkali solution and isolating a protein-containing polysaccharide from the substance. Thus their US—A—4051314 describes and claims a process for the production of a polysaccharide comprising forming a culture medium or a liquid extract of mycelium of one of certain *Coriolus* strains, separating said polysaccharide from the free proteins and other impurities contained in said culture medium or liquid extract and purifying said polysaccharide to produce a purified polysaccharide having an acute toxicity (LD$_{50}$) in mice of more than 20 g/kg for oral administration and more than 100 mg/kg for subcutaneous injection. Also, in their US—A—4202969 is disclosed a method of producing a nitrogen-containing polysaccharide, which method specifies:

(a) extracting *Coriolus versicolor* (Fr) Quél with an aqueous alkaline solution having a concentration of 0.01 to 2.0 N at 50° to 100°C;

(b) neutralising the resulting extract and including an alkaline salt therewith at a concentration of at least 0.4 M/l, whereby the molecular shape of said polysaccharide is rendered more spherical; and

(c) subjecting the thus-spherically modifed polysaccharide to ultrafiltration through a membrane as a molecular sieve under pressure of at least 0.5 kg/cm$^2$, whereby portions of the mixture having a molecular weight of less than 5000 are removed therefrom.

As a result of studying further the constituents of the substance extracted from the fungus, the present inventors have found a glycoprotein which has a higher protein moiety: saccharide moiety weight ratio and a specific bonding mode of amino acids in its protein moiety. The glycoprotein shows lectin-like physiological activities as well as an antitumour activity, quite different from conventional nitrogen-containing polysaccharides.

Accordingly, the present invention provides a glycoprotein having a molecular weight of from 5,000 to 300,000 as determined by the ultracentrifugation method; the ratio of the weight of its protein moiety, as determined by Lowry-Folin's method, to the weight of its saccharide moiety, as determined by the phenolsulfuric acid method, being from 50:50 to 80:20; the saccharide moiety containing fucose, ribose, arabinose, xylose, mannose, galactose, glucose and glucosamine, and the total weight of said xylose, said mannose and said glucose is more than 85% by weight of the total weight of said saccharides; the protein moiety containing aspartic acid, threonine, serine, glutamic acid, proline, glycine, alanine, cysteine, valine, methionine, cystathionine, isoleucine, leucine, tyrosine, phenylalanine, tryptophan, ornithine, lysine, histidine and arginine, and the total weight of said aspartic acid, said threonine, said serine, said glutamic acid, said glycine, said alanine, said phenylalanine, said valine, said leucine and said isoleucine is more than 75% by weight of the total weight of all of said amino acids; the amino acid at its N-end being tyrosine, leucine or alanine, the amino acid sequence at its C-end being leucine to phenylalanine to valine, the terminal amino acid being leucine; its elementary composition being from 35.2 to 49.3% of C, from 4.8 to 8.0% of H, from 4.3 to 12.3% of N, from a trace amount to 2.5% of S, from a trace amount to 1.2% of P and the balance being O; its isoelectric point being from pH 2.5 to pH 5.0; and having nucleic acid component. The glycoprotein can be used in the treatment of a tumour. The present invention also provides a process for producing a glycoprotein as referred to above, which process comprises extracting fruit bodies or mycelia of a basidiomycetous fungus belonging to the genus *Coriolus* with hot water or an aqueous 0.01 to 2.0N alkali solution, subjecting the resultant extract to dialysis and/or ultra-filtration thereby removing a low molecular weight substance(s) having a molecular weight of below 5,000, characterized in that the process further comprises collecting the fraction precipitating at a pH in the range of from 2.5 to 5.0 at the isoelectric point of the glycoprotein. The collection step can be achieved by fractionally collecting the fractions precipitating at the isoelectric point thereof of pH in the range of 2.5 to 5.0 by the method of fractional precipitation at the isoelectric point.

The present invention further provides a pharmaceutical composition which comprises a glycoprotein of the present invention as active ingredient together with a pharmaceutically acceptable carrier or diluent. The pharmaceutical composition can be in unit dosage form.

In the description which follows, reference will be made to the accompanying drawings in which:

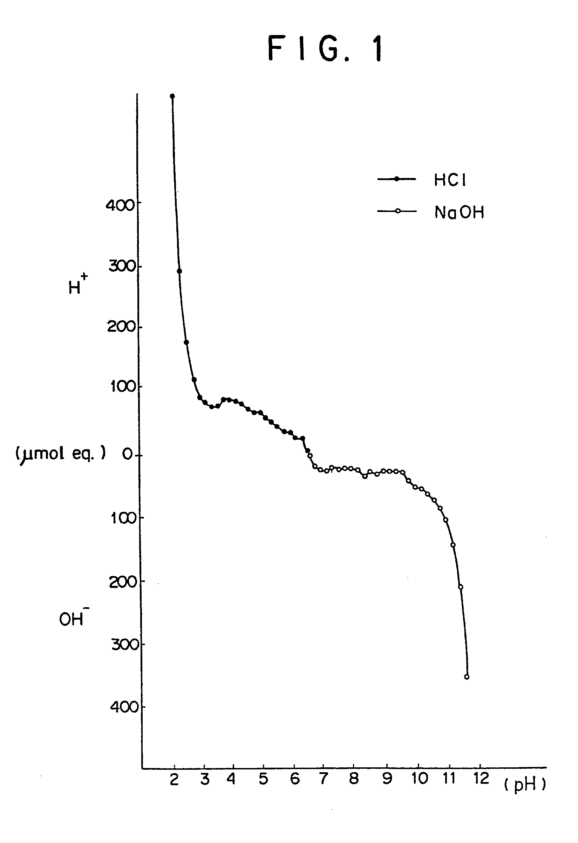

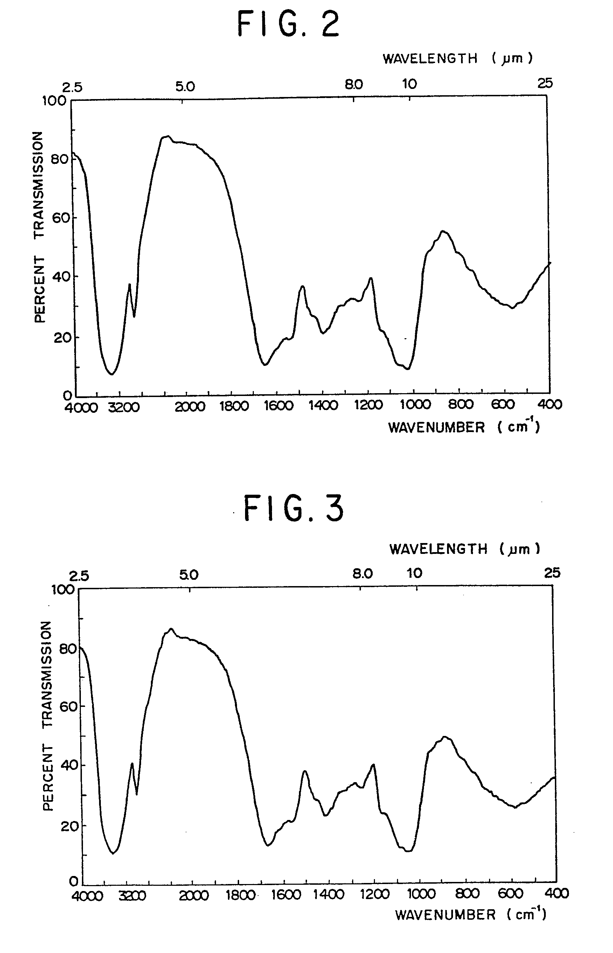

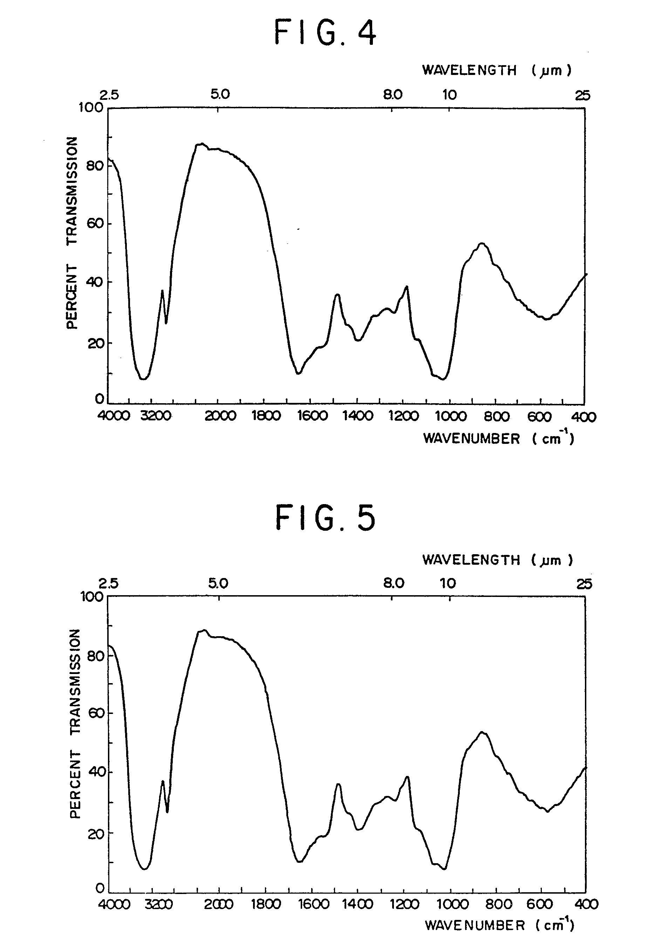

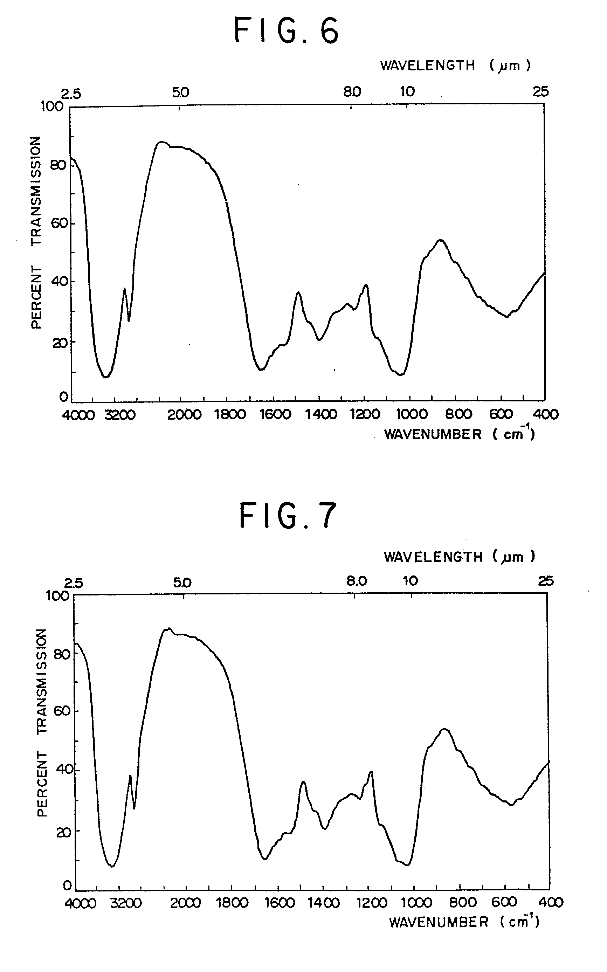

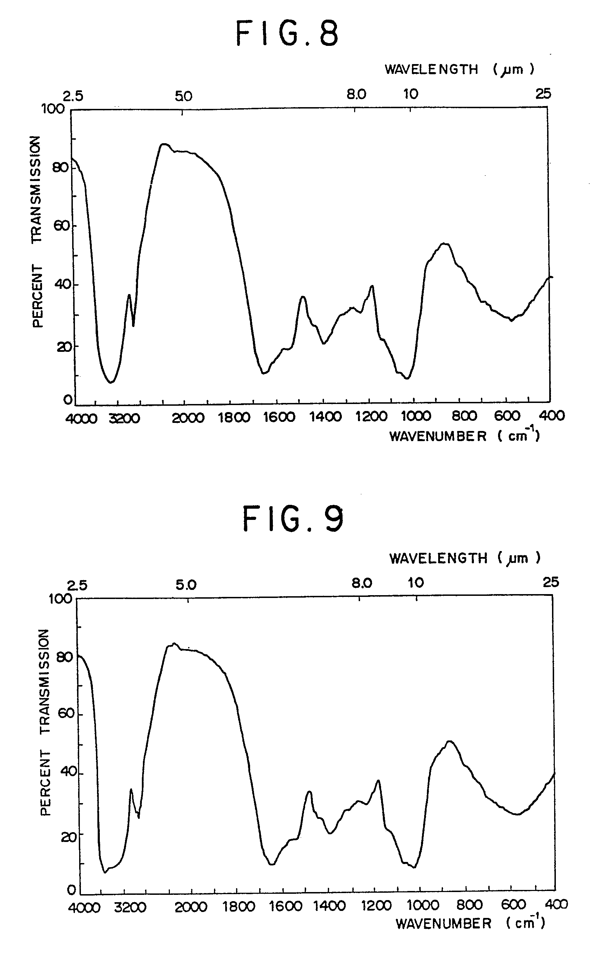

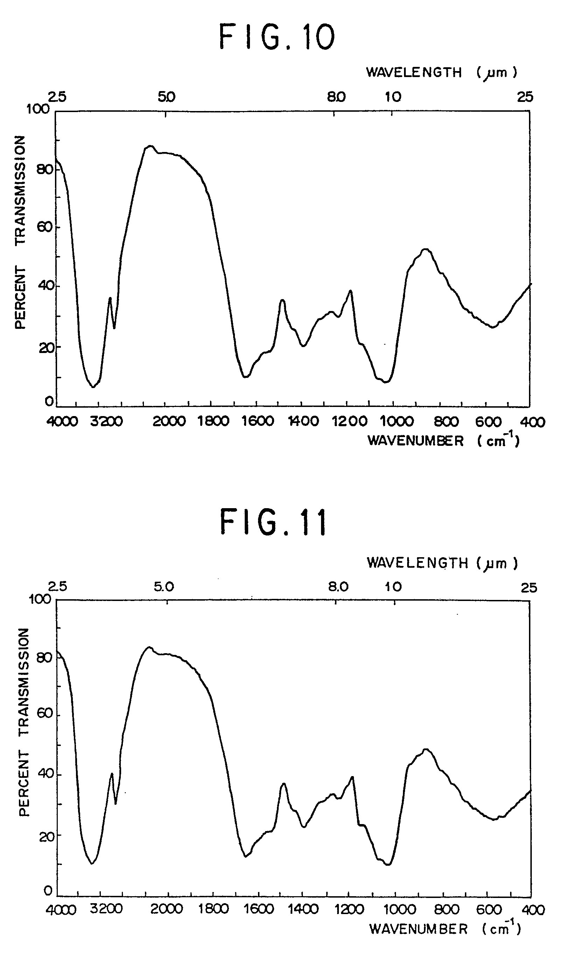

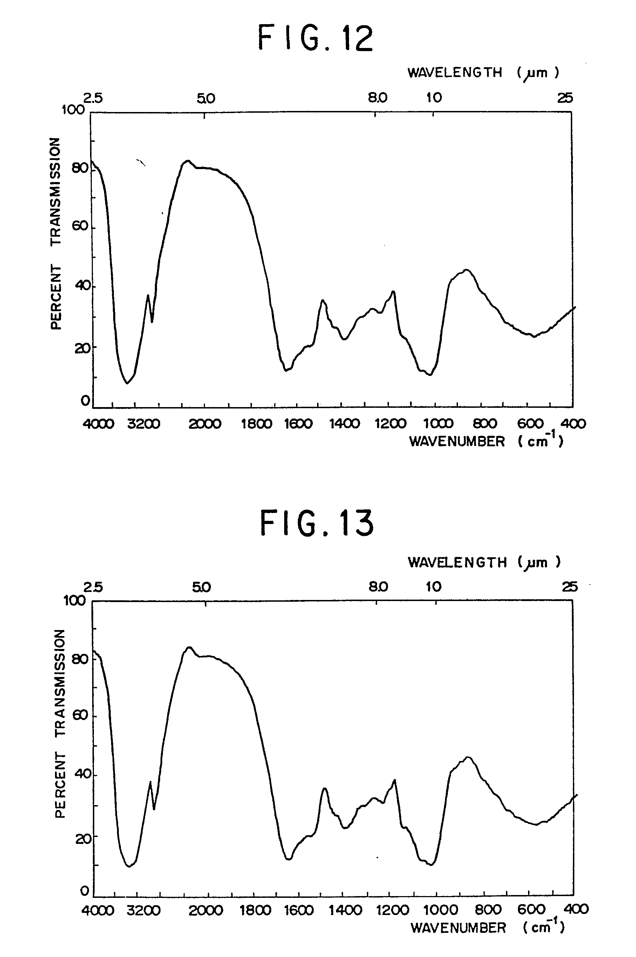

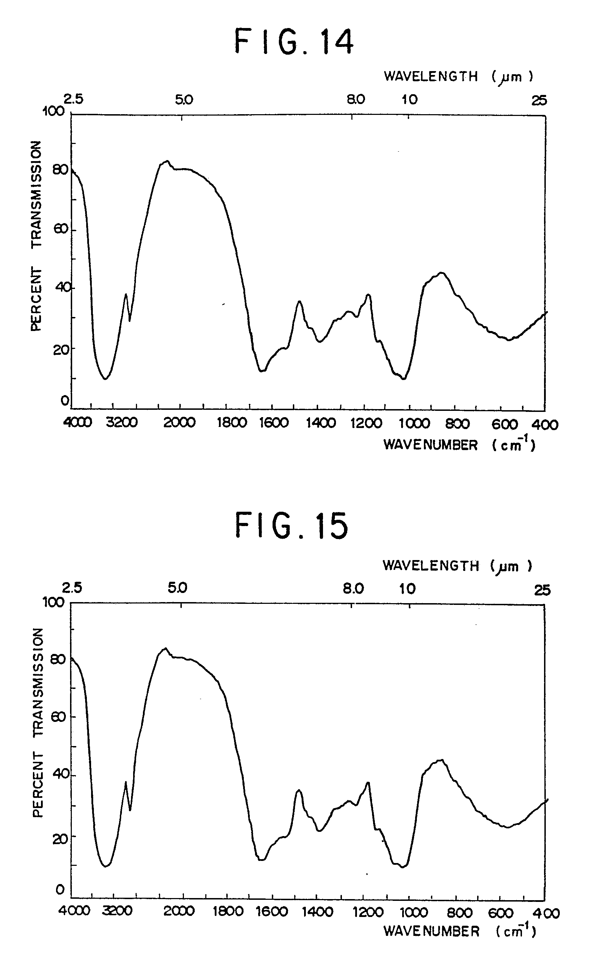

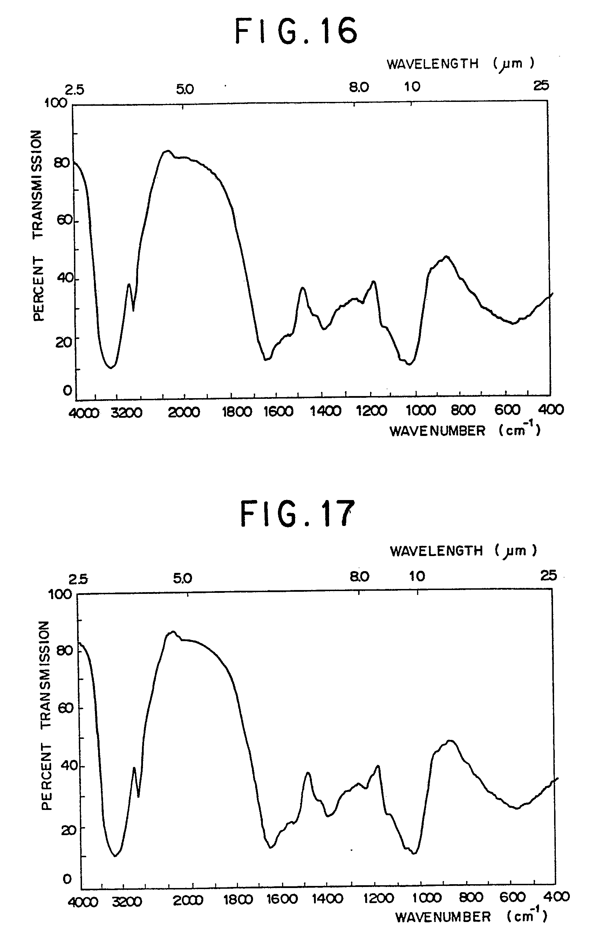

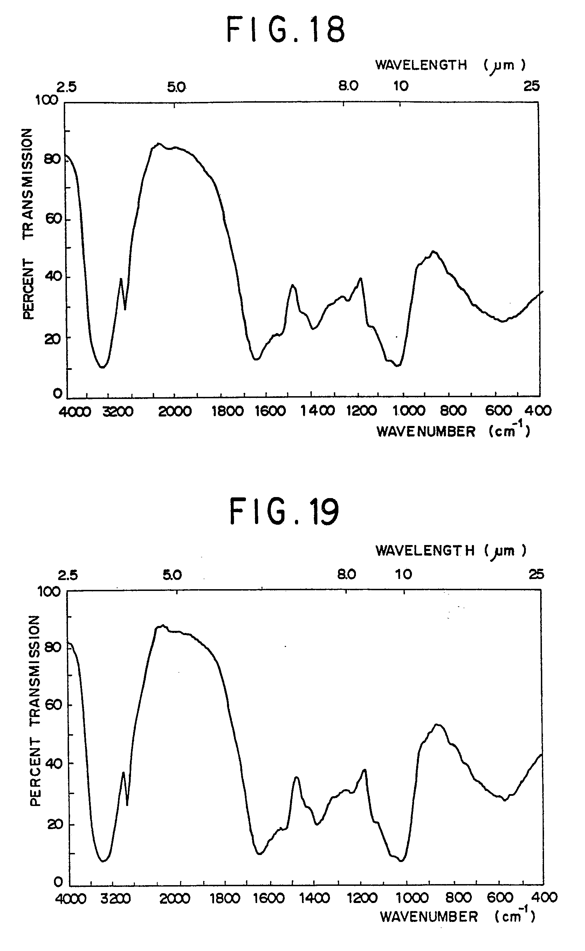

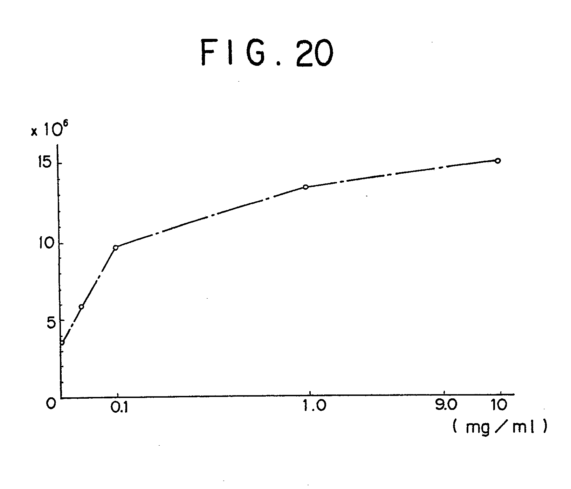

Figure 1 shows the titration curve (concentration of both H$^+$ and OH$^-$ against pH of a solution of the present substance) of a glycoprotein according to the present invention using an aqueous 0.02N hydrochloric acid solution and aqueous 0.02 sodium hydroxide solution; Figures 2—19 show the infrared absorption spectra of the glycoproteins according to the present invention of Examples 1 to 18 respectively; and Figure 20 shows the chemotaxis of macrophages to glycoproteins according to the present invention, based on the results of determination of the number of exuded cells against the concentration of the present substance in the peritoneal cavity by a hemocytometer.

A glycoprotein according to the present invention can be characterized and specified generically as follows:

(1) its molecular weight, as determined by the method of ultracentrifugation, is in a range of 5,000 to 300,000;

(2) the ratio of the weight of its protein moiety determined by Lowry-Folin's method, to the weight of its saccharide moiety, determined by the phenol-sulfuric acid method, is in a range of 50:50 to 80:20 (Lowry-Folin's method is described by O. H. Lowry et. al., J. Biol, Chem. Vol. 193, p. 265, 1951, and the phenol-sulfuric acid method is described in J. E. Hodge, "Methods in Carbohydrate-Chemistry" vol. 1, p. 388, 1962 ed. by R. L. Whistler, M. I. Wolfrom, Academic Press, New York, N. Y.).

(3) The amino acid occupying the N-end (terminal) of the protein moiety consists essentially of tyrosine, leucine or alanine;

(4) the amino acid sequence from the C-end (terminal) of the protein moiety is in the order of leucine to phenylalanine to valine, leucine being the terminal amino acid;

(5) its elementary composition consists essentially of 35.2 to 49.3% of C, 4.8 to 8.0% of H, 4.3 to 12.3% of N, a trace amount to 2.5% of S, a trace amount to 1.2% of P and the balance being O;

(6) its isoelectric point is from pH 2.5 to pH 5.0; and

(7) a nucleic acid is contained as a component,

(8) the composition of its saccharide moiety, as described above.

(9) The composition of its protein moiety, as described above.

A glycoprotein of the present invention (hereinafter referred to as the "present substance") can be prepared by the following process:

Fruit bodies or mycelia, preferably artificially cultured mycelia, of a basidiomycetous fungus, for example, *Coriolus versicolor* (fr.) Quél., *Coriolus hirsutus* (Fr.) Quél., *Coriolus pargamenus* (fr.) Pat., *Coriolus consors* (Fr.) Quél., *Coriolus pubescens* (Fr.) Quél., *Coriolus biformis* (Klotz) Pat. and *Coriolus conchifer* (Schw.) Pat. are extracted, as the starting material, with an aqueous solvent such as hot water or aqueous 0.01 to 2.0N alkali solution preferably at a temperature of 80 to 100°C for 1 to 8 hours. After removing the extraction residue, the aqueous extract is neutralized with an acid and condensed. After desalting the condensate, if necessary, the condensate is subjected to dialysis and/or ultrafiltration to remove a low molecular weight substance(s) having a molecular weight of below 5,000. The thus obtained product is condensed and dried to be a powdery substance, if necessary. Then, the product or the powdery substance can be treated with a method of fractional precipitation at isoelectric points of pH 2.5 to 5.0, preferably treating the medium containing the product or powdery substance under a pH of 2.5 to 5.0. and an ion strength of 0.1 to 3.1 $\mu$ at a temperature of 5 to 25°C for more than 0.5 hours. The fraction collected can be neutralised and subjected to dialysis or ultrafiltration.

Artificially cultured mycelia can be obtained by culturing a basidiomycetous fungus in a culture medium, homogenizing the mycelia which proliferate on the culture medium in the presence of an aqueous physiological saline solution and inoculating the thus homogenized culture medium containing the mycolium as a seed culture into stationary culture medium or submerged culture medium.

From the view point of productivity, the use of artificially cultured mycelia is preferable.

Table 1 below gives various deposited basidiomycetous fungi which can be used in the process of the present invention.

Of those, it is profitable from the view point of productivity to use the strain CM—101 of *Coriolus versicolor* (Fr.) Quél. (FERM-P 2412).

# O 058 093

## TABLE 1

| Species | Strain | Deposition number of the strain |
|---|---|---|
| *Coriolus versicolor* (Fr.) Quél. | CM—101 | FERM-P 2412 (ATCC 20547) |
| *Coriolus versicolor* (Fr.) Quél. | CM—102 | FERM-P 2413 (ATCC 20548) |
| *Coriolus versicolor* (Fr.) Quél. | CM—103 | FERM-P 2414 (ATCC 20545) |
| *Coriolus versicolor* (Fr.) Quél. | CM—104 | FERM-P 2415 (ATCC 20549) |
| *Coriolus versicolor* (Fr.) Quél. | CM—105 | FERM-P 2416 (ATCC 20550) |
| *Coriolus versicolor* (Fr.) Quél. | CM—106 | FERM-P 2417 (ATCC 20551) |
| *Coriolus versicolor* (Fr.) Quél. | CM—107 | FERM-P 2418 (ATCC 20552) |
| *Coriolus versicolor* (Fr.) Quél. | CM—108 | FERM-P 2419 (ATCC 20553) |
| *Coriolus versicolor* (Fr.) Quél. | CM—109 | FERM-P 2420 (ATCC 20554) |
| *Coriolus versicolor* (Fr.) Quél. | CM—110 | FERM-P 2421 (ATCC 20555) |
| *Coriolus versicolor* (Fr.) Quél. | CM—111 | FERM-P 2422 (ATCC 20556) |
| *Coriolus versicolor* (Fr.) Quél. | CM—112 | FERM-P 2423 (ATCC 20557) |
| *Coriolus versicolor* (Fr.) Quél. | CM—113 | FERM-P 2424 (ATCC 20558) |
| *Coriolus versicolor* (Fr.) Quél. | CM—114 | FERM-P 2425 (ATCC 20559) |
| *Coriolus versicolor* (Fr.) Quél. | CM—115 | FERM-P 2426 (ATCC 20560) |
| *Coriolus hirsutus* (Fr.) Quél. | CM—151 | FERM-P 2711 (ATCC 20561) |
| *Coriolus pargemenus* (Fr.) Pat. | CM—161 | FERM-P 2712 (ATCC 20562) |
| *Coriolus versicolor* (Fr.) Quél. | GX—101—3 | FERM-P 3686 (ATCC 20564) |
| *Coriolus consors* (Berk.) Imaz. | CM—166 | FERM-P 988 (ATCC 20565) |

(Note) FERM:Fermentation Research Institute, Agency of Industrial Science and Technology (Japan)
ATCC:American Type Culture Collection (U.S.A.)

The properties characterizing the thus obtained substance according to the present invention are more precisely described as follows:

(1) Molecular weight: 5,000 to 300,000.
The molecular weight value was obtained by determination via the method of ultracentrifugation generally used in determining the molecular weight of high polymeric substances.

(2) Colour reaction:
The presence of a proteinaceous structure was confirmed by the blue colouring in Lowry-Folin's reaction, and by the purplish blue colouring in the ninhydrin reaction on a hydrolyzate of the present substance with hydrochloric acid.
The presence of a saccharide structure was confirmed by purple colouring in the $\alpha$-naphthol-sulfuric acid reaction, brown colouring in the indole-sulfuric acid reaction, green colouring in the anthrone-sulfuric acid reaction, purplish brown colouring in the tryptophan-sulfuric acid reaction, greenish brown colouring both in the thioglycolic acid-sulfuric acid reaction and in the orcin-hydrochloric acid reaction.

4

(3) Weight ratio of the protein moiety to the saccharide moiety 50:50 to 80:20.

The respective contents of the protein moiety and the saccharide moiety of the present substance were quantitatively determinable by Lowry-Folin's method and the phenol-sulfuric acid method, and the weight ratio of both moieties can be calculated from the contents. In addition, since there was no observation of any precipitate both in Sevag's test method and the trifluoromethane test method, it was concluded that the protein moiety and the saccharide moiety are chemically bonded together.

(4) The mode of bonding between the saccharide and protein moieties:

Concerning the mode of bonding between the saccharide moiety and the protein moiety of the present substance, among the generally known modes of bonding between saccharide and protein such as N-acylglycosylamine type, C-glycoside type and glycoside ester type, etc., it was presumed that the N-acylglycosylamine type is predominant in the present substance because of the difficulty in breaking the bonding by the action of weak alkali and because of the confirmation of glucosamine on amino acid analysis of the hydrolyzate of the present substance.

(5) The amino acid composition of the protein moiety:

The amino acid composition of the protein moiety of the present substance, determined by the conventional method, is shown in Table 2:

TABLE 2

| Amino acid | Content in % by weight |
|---|---|
| Aspartic acid | 10 to 20 |
| Threonine | 4 to 6 |
| Serine | 3 to 6 |
| Glutamic acid | 10 to 16 |
| Proline | trace to 8 |
| Glycine | 6 to 10 |
| Alanine | 6 to 13 |
| Cystine | trace |
| Valine | 5 to 12 |
| Methionine | trace to 4 |
| Cystathionine | trace to 3 |
| Isoleucine | 3 to 8 |
| Leucine | 8 to 13 |
| Tyrosine | trace to 5 |
| Phenylalanine | 3 to 9 |
| Tryptophan | trace to 1 |
| Ornithine | trace to 2 |
| Lysine | 1 to 4 |
| Histidine | trace to 2 |
| Arginine | 1 to 4 |
| (Ammonia) | (1 to 7) |

As is seen in Table 2, of these amino acids, the total sum of the amounts of aspartic acid, threonine, serine, glutamic acid, phenylalanine, isoleucine, glycine, alanine, valine and leucine occupies more than 75% by weight of the total amino acids.

(6) The kinds of amino acid at the N-end of the protein moiety and the amino acid sequence from the C-end of the protein moiety:

The kinds of amino acid at the N-end of the protein moiety of the present substance were determined by the conventional method in which the amino group was dinitrophenylated, and after hydrolyzing the product with an acid, the thus formed dinitrophenylamino acid was identified by thin-layer chromatography and high speed-liquid chromatography to be tyrosine, leucine or alanine.

The amino acid sequence from the C-end of the protein moiety of the present invention was determined by using carboxypeptidase in which the amino acids obtained by hydrolyzing the present substance with carboxypeptidase were analyzed by thin-layer chromatography to be in the order of leucine-phenylalanine-valine.

(7) Isoelectric point of the present substance: pH 2.5 to 5.0.

In order to examine the physical specificity of the protein moiety of the present substance, the present substance was subjected to electrophoresis using the column ampholine method. In consideration of the isoelectric point of the present substance in a range of pH 2.5 to 5.0, it was confirmed that the present substance is an acidic glycoproteinaceous complex. In this connection, the titration curve of the present substance is as is seen in Figure 1 of the accompanying drawing. The curve is the result of titrating an aqueous 1% by weight solution of the present substance with aqueous 0.02 hydrochloric acid solution and aqueous 0.02N sulfuric acid solution.

(8) Constitution of the saccharide moiety of the present substance:

The constitution of the saccharide moiety of the present substance was confirmed following the ordinary method by which a hydrolyzate of the present substance was reduced and then acetylated, and the product was subjected to gas chromatographic analysis with the result shown in Table 3.

| (Mono) saccharide | % by weight |
| --- | --- |
| Fucose | 0.5 to 4.0 |
| Ribose | trace to 2.0 |
| Arabinose | 0.3 to 6.0 |
| Xylose | 2.0 to 40.0 |
| Mannose | 5.0 to 20.0 |
| Galactose | 0.5 to 8.0 |
| Glucose | 30.0 to 80.0 |
| Glucosamine | trace to 3.0 |

As are seen in Table 3, the saccharide moiety of the present substance contains eight kinds of monosaccharides, and although glucose predominates, xylose and mannose are also present in relatively large amounts. Consequently, the saccharide moiety of the present substance is a heteroglycane in which the sum of the percentages by weight of glucose, mannose and xylose is more than 85%.

(9) Presence of nucleic acid as a component:

Content of 0.01 to 0.50% by weight of the present substance of nucleic acid base.

After converting the organic phosphorus component of the present substance into inorganic orthophosphoric acid by the wet-ashing method, the orthophosphoric acid was determined by Fiske-Subbarow's method, and by subjecting the hydrolyzate of the present substance obtained by treatment with hydrochloric acid to high speed liquid chromatography using ultraviolet light at 254 nm, the presence of uracil as a nucleic acid base was confirmed. Confirmation of the presence of a nucleic acid in the present substance was achieved by ultraviolet absorption spectroscopy of the present substance. This revealed peaks at 260 nm and 280 nm with the ratio of extinction coefficients of 0.75 to 0.95.

In addition, following the Schmidt-Thannhauser-Schneider's method ordinarily used for fractionation of nucleic acids, fractionation of the nucleic acid contained in the present substance was attempted. However, since no nucleic acid could be fractionated, it is presumed that the nucleic acid in the present substance is present in a bonded state.

**0 058 093**

(10) Infrared absorption spectrum: refer to Figures 2 to 19 of the accompanying drawings.

The infrared absorption spectra of the specimens obtained in Examples 1 to 18 are shown in Figures 2 to 19 respectively. In these spectra, the broad absorption peak at 3600 to 3200 $cm^{-1}$ is considered to be OH groups which are involved in hydrogen bonding to various degrees because of the disappearance or the reduction of the intensity of this peak after O-methylation of the OH groups in the saccharide moiety of the present substance. The absorption peak at 1530 $cm^{-1}$ is attributable to the deformation vibration of the —NH group in the protein moiety of the present substance, and the broad adsorption peak at 1200 to 1000 $cm^{-1}$ is considered to be due to the asymmetrical stretching vibration of C—O—C bonding in the pyranose ring of saccharide moiety of the present substance.

(11) Appearance and solubility to solvents:

The present substance is a powdery material pale brown to brown in colour without any taste and odour, and is soluble in water but only sparingly soluble in methanol, pyridine, chloroform, benzene and hexane.

Physiological activities of the present substance are explained below:

(1) Acute toxicity: Acute toxicity to mice and rats.

The acute toxicity of the present substance to ICR-JCL mice 4 to 5 weeks old with a body weight of 21 to 24 g, and to Donryu rats 4 to 5 weeks old with a body weight of 100 to 150 g, was examined by intravenous, subcutaneous, intra-peritoneal or oral administration, observing their general symptoms, mortality and body weight during the 7 days after administration, and killing and conducting an autopsy. The results shown in Table 4 indicate that neither rats nor mice died even after administering the largest administerable amount of the present substance, making the calculation of an $LD_{50}$ impossible.

TABLE 4
Acute toxicity to mice and rats

| Animal species | Route of administration | $LD_{50}$ (mg/kg body weight) | |
| --- | --- | --- | --- |
| | | Female | Male |
| Mice | i.v. | >1300 | >1300 |
| | s.c. | >5000 | >5000 |
| | i.p. | >5000 | >5000 |
| | p.o. | >20000 | >20000 |
| Rats | i.v. | >600 | >600 |
| | s.c. | >5000 | >5000 |
| | i.p. | >5000 | >5000 |
| | p.o. | >20000 | >20000 |

(2) Lectin-like activity:

Substances showing so-called "lectin activity" have been found frequently in plant seeds, and "lectin activity" of a substance derived from a basidiomycetous fungus is disclosed in German democratic Republic's Patent No. 126,818. However, this patent does not refer at all to the "lectin activity" of a substance derived from a basidiomycetous fungus belonging to the genus *Coriolus*. The substance showing "lectin activity" found in a plant seed, for example concanavalin A obtainable from the seeds of *Canavalia ensiformis* and peanut lectin found in the seeds of *Arachis hypogaea*, differs from the present substance in the structure of amino acids in the protein moiety, and the weight ratio of the protein moiety to the saccharide moiety.

In consideration of the recent recognition of the importance of "lectin activity" in physiologically active agents, particularly in antitumour agents (anti-cancer drugs), it is significant that the present substance shows the following "lectin-like activities", which are the result of examination of the specific features of the "lectin-like activities" of the present substance, especially those considered to be important from the view point of utilization as a physiologically active agent:

(i) Test on the agglutinating activity to sarcoma-180 cells:

From the results of the test below it has been confirmed that the present substance agglutinizes sarcoma-180 cells.

7

Test method:

Cells of sarcoma-180 were intraperitoneally transplanted to an ICR-JCL mouse at $1 \times 10^6$ cells/animal, and on the 7th day after transplantation, the ascites were collected from the mouse.

After adding 2 to 5 times by volume of Hanks' solution to the ascites, the mixture was centrifuged for 2 min at 300 G and the supernatant solution was discarded. After collecting the precipitate and adding Hanks' solution to the precipitate, the mixture was centrifuged again for 2 min at 300 G and the supernatant solution was discarded. The precipitate was collected, and Hanks' solution was added to the precipitate to adjust the number of sarcoma-180 cells which proliferated in the thus obtained solution to $1 \times 10^6$/ml.

After placing 0.5 ml of the thus adjusted solution having the sarcoma-180 cells suspended therein and 0.5 ml of an aqueous physiological saline solution in which 3 mg of the present substance had been dissolved in a culture bottle and mixing the solutions well, the mixture was left standing for one hour at 37°C. Then, the supernatant solution was discarded, and the precipitate consisting mainly of sarcoma-180 cells was washed once with Hanks' solution. A portion of the thus washed cells was examined under a microscope to ascertain the number of agglutinated sarcoma-180 cells. As a control, the same procedures were repeated using 0.5 ml of the aqueous physiological solution not containing the present substance.

Test result:

A significant difference was recognized between the number of agglutinated lumps due to the present substance (120) and the number of those due to the control (20).

(ii) Test on the inhibition by a monosaccharide of agglutination of sarcoma-180 cells by the present substance:

A 0.5 ml specimen of sarcoma-180 cells suspended in 0.5 ml of Hanks' solution at a concentration of $1 \times 10^6$ cells/ml as in Test (i) and 0.5 ml of a physiological saline solution as in Test (i) containing 3 mg of the present substance and 1.5 mg of a monosaccharide, which had been incubated for 30 min. at 37°C, were poured into a tissue culture chamber and mixed together therein. After leaving the mixture to stand for one hour at 37°C, then discarding the supernatant medium and washing the residue with Hank's solution, the remaining residue was examined under a microscope to count the number of agglutinated lumps of cells.

Test results:

Of the tested monosaccharides, L-fucose and methyl $\alpha$-mannoside inhibited the agglutination of sarcoma-180 cells due to the present substance, as seen in the following results:

Results on the inhibitory function of a monosaccharide on the agglutination of sarcoma-180 cells by the present substance

| The present substance with the monosaccharide: | Number of agglu- tinated lumps | Inhibitory activity to agglutination |
|---|---|---|
| L-fucose | 27 | + |
| D-galactose | 118 | − |
| methyl $\alpha$-glucose | 115 | − |
| methyl $\alpha$-mannoside | 35 | + |
| N-acetylglucosamine | 85 | ± |
| L-rhamnose | 25 | + |
| The present substance only | 124 | |
| Blank test | 18 | |

(iii) Test on the inhibition of erythrocyte-agglutination:

25 Microlitres of an anti-human A-type erythrocyte serum or anti-human B-erythrocyte serum diluted to 16 times its original volume by an aqueous physiological saline solution and 25 microlitres of a solution of the substance of the present invention in an aqueous physiological saline solution were placed in a U-type microplate with 96 wells. After allowing the mixture to react for 30 min. at room

8

temperature, 25 microlitres of a 4% suspension of human A-type erythrocytes or of human B-type erythrocytes in an aqueous physiological saline solution was added to the reaction mixture to bring them into reaction for 3 hours at room temperatures. Then, the microplate was examined under a microscope to observe the state of erythrocyte-agglutination.

As a result, inhibition of blood type-specific erythrocyte agglutination by the substance of the present invention was recognized with a minimum inhibiting concentration of 0.48 mg/ml.

(iv) Test on blast transformation:
(a) Blast transformation of human lymphocytes:

Lymphocytes collected from peripheral blood of a healthy human adult were cultured for 5 days by the method of lymphocyte culture and the uptake of $^3$H-thymidine by the lymphocytes during the last 24 hours of the culture was measured. Blast transformation was determined from the extent of uptake of $^3$H-thymidine following the procedures below:

Venous blood taken from a healthy adult was placed on Ficoll-Conray's liquid for separation by specific gravity, and the layers were subjected to centrifugation for 30 min. at 400 G to obtain the lymphocytes, which were suspended in RPMI—1640 culture medium to which 20% fresh human AB-type serum has been added, at a concentration of $7.5 \times 10^5$ cells/ml. Into each well of a microplate (Model Microtest II, made by Falcon Company), 0.2 ml of the thus prepared lymphocyte suspension was poured. Aqueous physiological saline solution containing the present substance was poured into each well to make the final concentration of the present substance in the solution in each well 0.1 mg/ml. After keeping the microplate in an incubator at 37°C for 4 days under an atmosphere of air containing 5% of carbon dioxide, 0.05 micro-Ci of $^3$H-thymidine was added into each well of the microplate and the microplate was kept for an additional 24 hours in the incubator under the same conditions as above. The thus treated lymphocytes were harvested using a cell harvester (Model MASH II, made by Labo Science Company) on glass fiber. After drying the glass fiber well, it was taken to a counting vial, and after adding 3 ml of Liquid Scintillation Cocktail (PCS, made by Amersham Company) into each well, the radiation activity as a result of $^3$H-thymidine uptake by the lymphocytes was determined by the scintillation counter, the units being counts per min (c.p.m.).

Test results

The results of the test showed that activity of the present substance causing blast transformation corresponded to 8,000 to 10,000 c.p.m. of uptake of $^3$H-thymidine by the addition of 0.1 mg of the present substance/ml as compared to 100 to 2,000 c.p.m. of uptake of $^3$H-thymidine by a control (physiological saline solution not containing the present substance). The activity of the present substance is significant over the control at the level of $P < 0.01$.

(b) Blast transformation of guinea-pig lymphocyte:

Spleen lymphocytes of a guinea pig ($1 \times 10^6$ cells/ml) and the present substance (50 micrograms/ml) were kept for 4 days at 37°C under an atmosphere of air containing 5% of carbon dioxide. After adding 0.5 micro-Ci of $^3$H-thymidine, the mixture was kept for an additional 24 hours under the same conditions. By determining the uptake of $^3$H-thymidine as in (a), the stimulation index (S.I.) of the present substance was calculated from the following formula:

$$\text{Stimulation index (S.I.)} = \frac{\text{c.p.m. of the test group}}{\text{c.p.m. of control group 1)}}$$

wherein 1) did not contain the present substance.

As a result, it was found that the stimulation index of the present substance was significantly larger than 1, the fact indicating that the present substance does cause blast transformation.

The phenomenon of blast transformation shown above due to the present substance is a phenomenon which necessarily occurs when lymphocytes are stimulated by an antigen to differentiate into functional cells such as antibody-producing cells, etc. In addition, as a result of blast transformation, i.e. a result of activation of cell division, an increase in the number of cells of the cell group which has a reactivity to the antigen is brought about (clonal expansion). A portion of these cells return to the rest state, and these resting cells become the main body of "immunological effect" in the case where they are re-subjected to the stimulation of the same antibody as contrasted to the case of first stimulation. In this manner, the phenomenon of blast transformation is a phenomenon having a very important meaning in the immunoresponse. The activity of the present substance in stimulating blast transformation is non-specific, that is not only a specified cell group but also cell groups in a broader range are stimulated by the present substance. It is considered that such an activity brings about a non-specific immunoactivating effect and contributes to the strengthening of host resistance against microbial infection and cancers. Actually, it has been known that activity of causing blast transformation is observed in immunotherapeutics such as BCG and OK—432 against cancers, which act via non-specific immunoactivation. As a result of examining antitumour activity of orally administered phytohemagglutinin, concanavalin A, which has been known as a representative lectin, against sarcoma-180, 30 to 50% antitumour activity was confirmed. This finding suggests a connection between activity causing blast transformation and antitumour activity.

(v) Affinity of the present substance to the surface of a lymphocyte:

The specific activity of the present substance to bind with a lymphocyte has been confirmed as follows using Fluorescence Activated Cell Sorter II (FACS II, made by Beston-Dickinson Company, USA).

A thymus of a C57BL/6 mouse was dissociated in a phosphate buffer to obtain a single-cell suspension, which was then treated with a Tris-hydrochloric acid buffer containing 0.83% by weight of dissolved ammonium chloride to remove erythrocytes from the suspension and obtain an aqueous suspension of thymocytes. Separately, 0.6 mg of fluorescein iso-thiocyanate (FITC) was added to 0.4 ml of a 5% by weight solution of the present substance in the phosphate buffer of pH of 7.0. After stirring the mixture overnight at 4°C, the mixture was applied onto a dextran gel to remove FITC which was not bonded to the present substance and to obtain an aqueous solution of the present substance (in the phosphate buffer) labelled with FITC.

After bringing $1 \times 10^7$ thymocyte cells of a C57BL/6 mouse and 0.1 ml of a phosphate buffer solution containing the present substance labelled with FITC into reaction for 20 min. at 4°C, the thus reacted lymphocytes were analyzed by FACS (loc. cit.). The strong fluorescence that was observed of the lymphocytes after reaction with the FITC-labelled substance of the present invention confirmed that the present substance binds to lymphocytes.

Further, it has also been confirmed that an immunosuppressive factor, obtained from the ascites of an ICR-JCL mouse bearing Ehrlich carcinoma according to the method of Motoki et al. (Gann, Vol. 66(5), 569—572, 1975), binds to lymphocytes.

However, in the case where the present substance not labelled with FITC was reacted with the lymphocytes at 4°C for 20 min., and the reaction mixture was further reacted with the FITC-labelled immunosuppressive factor mentioned above, the strength of fluorescence of the thus reacted lymphocytes was weaker than that of lymphocyte with which the present substance had not been reacted. From these results, it is considered that the present substance inhibits the binding of the immunosuppressive factor to lymphocytes.

The fact that the present substance binds to lymphocytes has been confirmed also by the examination under an electron microscope as follows:

After reacting 25 mg of the present substance with 25 mg of ferritin, the reaction product was reacted in vitro with thymocytes of a C57BL/6 mouse.

In the case where only ferritin was brought into reaction with thymocytes, the ferritin did not bind to the surface of the thymocytes. However, where the present substance had been reacted with the ferritin, the binding of the present substance to the surface of the thymocytes could be observed under a scanning electron microscope.

Such binding of the present substance to the thymocytes is considered to correspond to the antitumour effect and immunoresponse of the present substance as described as follows:

In the case where an anti-tumour substance stimulates lymphocytes to cause blast transformation, the binding of the substance to the lymphocytes is considered to be the first step of blast transformation. However, not only a mechanism by which the antitumour effect of the substance is exhibited via the activation of lymphocytes but also another mechanism is considered. In this latter mechanism the substance suppresses the binding of a principle which inhibits the activation of lymphocytes to lymphocytes.

Namely, as shown above, the presence of an immunosuppressive factor in the ascites of a cancer-bearing individual, for example an ICR-JCL mouse bearing Ehrlich cancer, has been known. It has been found by the present inventors that the present substance inhibits the binding of this immunosuppressive factor to lymphocytes, thus leading to the release of the suppression of the function of lymphocyte by the immunosuppressive factor.

Thus it is considered that the present substance, on one side, directly activates the lymphocytes and, on the other side, inhibits the binding of the immunosuppressive factor to the lymphocytes to exhibit an antitumour effect and anti-infection effect.

(vi) On the chemotaxis of macrophage to the present substance:

To respective groups of C57BL/6-female mice, 1 ml/animal of aqueous physiological saline solutions containing 0.03, 0.1, 1.0 or 10 mg of the present substance per ml was administered intraperitoneally. The mice in one control group were only administered with 1 ml each of aqueous physiological saline solution. 48 Hours after administration, all mice were subjected to venesection under ether anesthesia and 4 ml of heparinadded Hanks' solution was intraperitoneally injected into each mouse. After massaging the abdominal cavity of the mouse well, the injected solution was collected with a Komagome pipette, and the macrophage cells exuded into the collected solution were counted by a hemocytometer. The results is shown in Figure 20 of the accompanying drawings, wherein the number of exuded macrophage cells is taken as the ordinate and the concentration of the present substance is taken as the abscissa. As is seen in Figure 20, the number of macrophage cells showed a tendency to rise with an increase in the concentration of the present substance. This phenomenon can be interpreted as showing that the present substance promotes the chemotaxis of macrophages.

It is known that macrophages contribute to the resistance of a host to cancer and to infection due to the macrophage's own phagocytosis and antibody-dependent cytotoxic function. It is also known that macrophages are deeply connected to the so-called immuno-system with a result of antigen-presentation and adjustment of immunoreaction. Moreover, in the macrophages stimulated by the present substance the functions of the macrophages have been enhanced as mentioned above. Since the raised chemotaxis is an index of macrophage activation, it is presumed that the present substance also reinforces the host resistance attributable to macrophages via their activation.

(e) Antitumour activity of the present substance:
(i) Antitumour activity to solid-type sarcoma-180 tumour:

Cells of sarcoma-180 proliferated by the ordinary method were transplanted into the abdominal cavity of 4 groups of ICR-JCL mice at $1 \times 10^6$ cells/animal. 24 Hours after transplantation, to each of 3 groups of the mice, one of the present substances was administered intraperitoneally once very other day a total of 10 times at respective dosages of 1, 10 and 100 mg/kg per time. 25 Days after transplantation, the tumours appearing in all the mice were extirpated. The average weight of tumours of each group to which a present substance had been administered (T) was compared with the average weight of tumours of a fourth group of mice to which the present substance was not administered (C) according to the following formula, and the results were expressed as the tumour-inhibiting extent:

$$\text{Tumour-inhibiting extent(\%)} = (1 - \frac{T}{C}) \times 100.$$

Furthermore, in another test, the present substance was orally administered, instead of by intraperitoneal injection, to two groups of tumour-transplanted mice at the respective dosages of 50, 100 and 250 mg/kg/time, and the tumour-inhibition was examined in the same manner as above.

(ii) Antitumour activity to Ehrlich ascites tumour:

After incubating $1 \times 10^6$ cells of Ehrlich ascites tumour (hereinafter refer to as EC cells) in Hanks' solution containing 5 mg of one of the present substances for 3 hours at 37°C, the thus incubated cells were transplanted into the abdominal cavity of ICR-JCL mouse (10 animals per group) at $1 \times 10^6$ cells/animal. The mortality of the treated mice was observed for 20 days after transplantation to find the survival rate of the mice and the antitumour activity of the present substance administered at 50 mg/kg as mentioned above, the body weight of the mouse being 20 g on average. All mice in a control group to which tumour cells were transplanted but to which the present substance was not administered died within 20 days of transplantation due to the tumour.

The results of the tests (i) and (ii), shown in Table 5 below, verify the anti-tumour activity of the present substance.

Since the present substance shows a noted antitumour activity, it is useful as an antitumour agent. It can be orally administered. In addition, since the present substance has lectin-like physiological activity, it has an immunomodulating activity on a host, and not only for cancer-bearing patients but also for all patients it exhibits a prominent effectiveness in activating the immune system and preventing infectious diseases due to viruses and bacteria.

Although the present substance will ordinarily be administered orally to patients in a powdery state, depending upon the condition of the patient it may be intraperitoneally administered by injection. The oral dosage of the present substance is ordinarily 1 to 100 mg/kg body weight/day. It exhibits an excellent tumour-inhibiting effect at very low doses compared to hitherto reported glycoproteinaceous substances and polysaccharides derived from a basidiomycetous fungus.

The following Examples illustrate the present invention.

Example 1
Preparation of a glycoprotein

Strain CM—101 of *Coriolus versicolor* (Fr.) Quel. [FERM-P 2413, (ATCC 20547)] was cultured for ten days in a culture medium comprising 5% by weight of glucose, 0.2% by weight of peptone, 0.3% by weight of yeast extract, 0.1% by weight of $KH_2PO_4$ and 0.1% by weight of $MgSO_4 . 7H_2O$. The mycelia which developed on the surface of the culture medium was homogenized with an aqueous physiological solution for use as a seed culture. Portions of the seed culture were inoculated into respective 200 ml amounts of the same culture medium as above in 100 culture bottles of 1 litre capacity, and cultured for 25 days at 25 to 27°C. The mycelia propagated were obtained in a yield of 2.0 to 4.5 g per culture bottle.

One hundred grams of the thus obtained mycelia was extracted with 3 litres of aqueous 0.1N

sodium hydroxide solution at 97°C for one hour. After removing the extract residue, the thus obtained extract was neutralized with an acid and condensed.

Subsequently, the condensate was treated by dialysis and ultra-filtration to remove substances with a molecular weight below 5,000, desalting having been carried out before dialysis and ultra-filtration. The thus treated condensate from which the low-molecular weight substances have been removed (or the powdery substance obtained by drying the thus treated condensate) was mixed with a phosphate buffer to maintain the pH of the mixture at 3.5. After neutralizing the supernantant solution obtained by centrifugation of the mixture, the neutralized supernatant was subjected to ultrafiltration under 147 kPa (1.5 kg/cm²) and at a temperature of 10°C while stirring, cooling and utilizing an ultrafiltration apparatus (Model: Table-mount Highflow-2000, made by Amicon Company, with a diaphragm of grade DM—5) to remove the salts in the supernatant. After condensing the desalted supernatant, a phosphate buffer was added to the condensate to maintain the pH of the resultant mixture at 3 and the mixture was centrifuged to collect the precipitate.

The above-mentioned series of procedures by which the fractions precipitating at a stabilized pH of 3.5 under the condition of the ion strength 0.30$\mu$ at a temperature of 5°C for 2 hours were discarded and then only the fractions precipitating from the remaining solution after maintaining the pH at 3.0 thereof under the same condition were collected comprises a method of fractionation by precipitation at an isoelectric point within a range of pH 3.0 to 3.5.

The thus obtained precipitate was re-dissolved in water while adjusting the pH of the thus obtained solution to 7, and was desalted by the method above to purify the solution. The desalted precipitate was then dried and the present substance was obtained in a powdery state.

Molecular weight, elementary analytical data, colour reactions of sugars and proteins, specific rotatory power, infrared absorption spectrum, results of amino acid analysis, analytical data on N-end and C-end amino acids, sugar components of the saccharide moiety, respective contents of protein- and sugar moiety, ultraviolet absorption spectrum, respective contents of uracil and organic phosphorus both of which show the presence of nucleic acid and specific physiological properties of the present substance are shown in Table 5. In Table 5 "t" means "trace". The methods used to determine these properties were as follows:—

Molecular weight

After preparing a 0.3% by weight solution of the present substance by dissolving it into an aqueous 0.1M potassium chloride solution, the sedimentation velocity of the present substance was determined at 25°C for 5 hours at a liquid column height of 1.7 mm in an ultra high speed centrifuge at 22,000 r.p.m. The molecular weight of the present substance was obtained by the usual calculation.

Specific rotatory power

The rotatory power of an aqueous 0.1% by weight solution of the present substance to the D line of sodium (589 nm) was determined in a cell of 5 cm length. The value was calculated to obtain the specific rotatory power of the present substance.

Amino acid analysis

In a glass tube, 10 mg of the present substance was placed as a specimen. After adding 4 ml of 6N hydrochloric acid solution and freezing the contents with a dry-ice and acetone mixture, the tube was sealed under reduced pressure and then heated for 24 hours at 110°C to hydrolyze the substance. After collecting and drying the reaction product, the dried matter was dissolved in 30 to 40 ml of a citrate buffer of pH 2.2. The solution was fed to apparatus which effects an amino acid analysis.

Analytical data on the amino acid at the N-end

While using 7 mg of the present substance as a specimen, analytical data on the amino acid at the N-end of the present substance was determined by the DNP (dinitrofluorobenzene) method with trimethylamine as follows:

After dissolving 7 mg of the specimen in 0.7 ml of water, 0.14 ml of an aqueous 5% by weight solution of trimethylamine and 0.7 ml of an ethanolic 5% by weight solution of dinitrofluorobenzene were added to the solution. The mixture was stirred for 3 hours at 28°C in the shade. A few drops of water and a small amount of an aqueous solution of trimethylamine was added to the mixture which was then washed four times with diethyl ether. A few drops of concentrated hydrochloric acid were then added to the aqueous layer. After further washing the aqueous acidic layer with ether three times, the acidity of the aqueous layer was adjusted to that of 6N hydrochloric acid and heated for 20 hours at 110°C to effect hydrolysis. After adding water to the thus obtained hydrolyzate to bring the acidity of the hydrolyzate to that of about 1N hydrochloric acid, the mixture was extracted three times with diethyl ether. The ether layer was dried and then used as the specimen for analysis by high-speed liquid chromatography (with a column of Zorbax O.D.S. 4.6 mm in diameter and 250 mm in length) while eluting with a gradient eluting system of acetonitrile- sodium dihydrogen phosphate and using 254 nm ultraviolet light for detection.

12

Analysis of amino acid at the C-end

A solution was obtained by dissolving 250 mg of the present substance in 9.46 ml of 0.0025M Tris-buffer of pH 8.6. 0.54 ml of an aqueous solution of 0.3 units of carboxypeptidase-DFP (made by SIGMA Company) was added to the solution to bring the total volume of the mixture to 10 ml. While heating the thus obtained mixture at a constant temperature of 30°C each 1 ml of the respective specimens was fractionally collected at predetermined intervals, and 1.5 ml of an aqueous 50% by weight solution of acetic acid was added to each specimen to adjust the pH to 2 to stop the enzymatic reaction. The thus sedimented precipitate was removed by centrifugation. The resultant supernatant was collected, dried and dissolved in a buffer of pH 2.2, and then subjected to an automatic amino acid analyzer to determine the free amino acid.

Analysis of sugar components of saccharide moiety

In a glass ampoule, 10 mg of the present substance was placed. After adding 1.0 ml of an aqueous 1N sulfuric acid solution into the ampoule, the mixture was heated at 100°C for 16 hours to effect hydrolysis. After neutralizing the hydrolyzate at room temperature with barium carbonate and removing the thus sedimented precipitate by filtration, the filtrate was reduced by the addition of sodium boron hydride and the reactant was de-salted with an ion-exchanging resin. After condensing the de-salted solution to dryness and removing the remaining boron compound under a reduced pressure with methanol by azeotropic distillation, the residue was dried to solid. The residual solid was dissolved in pyridine and then acetylated with acetic anhydride at 100°C. After maintaining the thus obtained reaction mixture under a reduced pressure to remove the reagent remaining in the reaction mixture containing acetylated products, the residue was dissolved in carbon tetrachloride and subjected to gas-chromatograph to obtain the acetylated sugars fractionally.

Determination of uracil as a base of nucleic acid

After hydrolyzing the present substance with hydrochloric acid, the hydrolyzate was subjected to high-speed liquid chromatography with a Solvacks CN column while using aqueous 6% by weight solution of acetic acid as the moving phase and examining the eluent with ultraviolet light of 254 nm to detect the peak due to uracil.

Quantitative determination of organic phosphorus for confirming the presence of nucleic acid in the present substance

After decomposing organophosphorus components of the present substance into inorganic orthophosphoric acid by the wet-ashing method, the resultant phosphoric acid was quantitatively determined. Namely, 2 ml of an aqueous solution containing 20 mg of the present substance and 2 ml of aqueous 5N sulphuric acid solution were heated in a Kieldahl decomposition flask until the content turned brown in colour. Then after cooling, one or two drops of aqueous 30% by weight solution of hydrogen peroxide were added to the contents of the flask. The flask was again heated until the contents became colourless. After cooling the flask, one ml of pure water was added to the contents of the flask. After heating the flask for 5 min. in a hot-water bath and adjusting the volume of the contents to a predetermined value, a part of the contents was subjected to Fiske-Subbrow's method to obtain the amount of orthophosphoric acid in the decomposed specimen of the present substance.

Examples 2 to 18

Instead of strain CM—101 of *Coriolus versicolor* (Fr.) Quel. (FERM-P 2412, ATCC 20547), each of the respective strains of the fungi shown in Table 1 was used as the starting material to obtain a glycoprotein of the invention by the same procedures as in Example 1, except for carrying out the fractionation by precipitation at an isoelectric point in the pH ranges shown in Table 5 rather than at from pH 3.0 to 3.5. The properties of the substances obtained in Examples 2 to 18 were determined by the same methods as in Example 1 and are also shown in Table 5 and thereafter. In Table 5 "t" means "trace".

13

TABLE 5
Physical and physiological properties of the present substance

| Item \ Example No. | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| Number of deposition FERM-P: | 2412 | 2413 | 2414 | 2415 | 2416 | 2417 | 2418 | 2419 | 2420 | 2421 | 2422 | 2423 | 2424 | 2425 | 2426 | 2711 | 2712 | 988 |
| Range of pH in precipitation at isoelectric point | 3.0—3.5 | 3.5—4.0 | 4.0—4.5 | 3.0—4.0 | 3.0—4.5 | 2.5—3.0 | 2.5—3.5 | 2.5—4.0 | 2.5—4.5 | 2.5—5.0 | 4.5—5.0 | 3.5—5.0 | 2.5—4.5 | 3.0—5.0 | 2.5—4.5 | 2.5—5.0 | 2.5—5.0 | 2.5—4.5 |
| Molecular weight range ($\times 10^4$) | 0.5—30 | 0.5—30 | 0.5—30 | 0.5—30 | 0.5—30 | 0.5—30 | 0.5—30 | 0.5—30 | 0.5—30 | 0.5—30 | 0.5—30 | 0.5—30 | 0.5—30 | 0.5—30 | 0.5—30 | 0.5—30 | 0.5—30 | 0.5—30 |
| Elementary analytical data (%)  C: | 46.0 | 47.2 | 40.5 | 44.3 | 48.5 | 39.8 | 44.1 | 42.6 | 38.5 | 40.9 | 41.8 | 37.6 | 43.3 | 42.6 | 40.1 | 45.4 | 42.7 | 38.3 |
| H: | 6.2 | 7.8 | 5.3 | 6.9 | 5.5 | 6.3 | 7.0 | 6.2 | 6.2 | 5.8 | 6.4 | 7.2 | 5.8 | 7.0 | 7.5 | 5.0 | 5.8 | 6.5 |
| N: | 10.2 | 6.0 | 6.9 | 7.5 | 10.3 | 12.1 | 7.0 | 8.8 | 7.0 | 5.5 | 4.5 | 6.8 | 7.3 | 9.2 | 9.9 | 11.6 | 10.1 | 7.6 |
| S: | t | 0.6 | 0.3 | 2.3 | t | 0.5 | 1.5 | 1.8 | 0.8 | t | 1.2 | 1.3 | 0.9 | 0.5 | 1.8 | 2.0 | t | 0.8 |
| P: | 0.7 | 0.8 | 0.5 | 0.6 | 0.3 | 1.0 | 0.2 | t | t | 0.7 | 0.2 | 0.4 | 0.1 | 0.6 | t | 0.3 | 0.7 | 0.5 |
| O: | 36.9 | 37.6 | 46.5 | 38.4 | 35.4 | 40.3 | 59.8 | 40.6 | 47.5 | 47.1 | 45.9 | 46.7 | 42.6 | 40.1 | 40.7 | 35.7 | 40.7 | 46.3 |
| Content of saccharide (% by weight) | 32.2 | 43.2 | 41.9 | 44.7 | 32.5 | 24.9 | 30.7 | 34.0 | 37.9 | 46.0 | 49.7 | 41.6 | 40.2 | 42.9 | 40.7 | 37.8 | 35.3 | 40.1 |
| Content of protein (% by weight) | 67.8 | 57.8 | 58.1 | 55.3 | 67.5 | 75.1 | 69.3 | 66.0 | 62.1 | 54.0 | 50.3 | 58.4 | 59.8 | 57.1 | 59.3 | 62.2 | 64.7 | 59.9 |

0 058 093

TABLE 5 (Continued)

| Item | Example No. | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|---|
| Saccharide component and its content (% by weight) | fucose | 1.1 | 3.9 | 2.1 | 0.6 | 1.8 | 2.3 | 2.7 | 2.6 | 2.9 | 2.0 | 1.2 | 2.8 | 2.0 | 2.7 | 1.8 | 3.6 | 2.9 | 1.5 |
| | ribose | 1.0 | t | t | 1.8 | 1.5 | 0.8 | t | 0.9 | 1.6 | 1.1 | t | t | 1.3 | 1.0 | 1.2 | t | 1.0 | 1.3 |
| | arabinose | 5.3 | 1.9 | 2.3 | 0.3 | 0.5 | 2.6 | 0.9 | 4.7 | 1.5 | 0.8 | 0.5 | 1.9 | 3.4 | 0.6 | 0.4 | 1.8 | 1.6 | 1.9 |
| | xylose (1) | 13.5 | 34.9 | 19.5 | 23.8 | 23.5 | 31.0 | 30.0 | 32.4 | 22.6 | 20.2 | 19.8 | 21.7 | 31.6 | 28.3 | 22.5 | 18.2 | 19.3 | 16.5 |
| | mannose (2) | 10.6 | 13.2 | 17.0 | 16.3 | 11.3 | 12.4 | 13.9 | 13.6 | 15.1 | 14.1 | 12.3 | 11.4 | 10.9 | 12.5 | 14.0 | 13.5 | 11.5 | 15.0 |
| | galactose | 2.0 | 4.1 | 6.2 | 3.5 | 3.1 | 2.9 | 2.8 | 2.0 | 2.6 | 2.4 | 5.8 | 4.1 | 3.5 | 2.8 | 2.5 | 2.6 | 2.7 | 4.0 |
| | glucose (3) | 66.5 | 42.0 | 52.9 | 53.7 | 58.3 | 48.0 | 49.7 | 43.6 | 53.7 | 59.6 | 61.4 | 58.1 | 46.3 | 52.1 | 57.6 | 61.3 | 61.0 | 60.8 |
| | glucosamine | t | t | t | t | t | t | t | 0.2 | t | t | t | t | 1.0 | t | t | t | t | t |
| | Sum of (1), (2) and (3) | 90.6 | 90.1 | 89.4 | 93.8 | 93.1 | 91.4 | 93.6 | 89.6 | 91.4 | 93.9 | 93.5 | 91.2 | 88.8 | 92.9 | 94.1 | 93.0 | 91.8 | 92.3 |

0 058 093

15

TABLE 5 (Continued)

| Item | Example No. | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|---|
| Amino acid component (% by weight) | aspartic acid | 14.6 | 10.8 | 12.6 | 14.9 | 12.2 | 13.0 | 13.9 | 14.0 | 13.5 | 15.3 | 12.0 | 16.0 | 10.7 | 11.6 | 13.1 | 14.7 | 14.4 | 13.1 |
| | threonine | 4.1 | 6.0 | 4.9 | 5.8 | 4.2 | 4.4 | 4.1 | 4.8 | 5.2 | 4.9 | 4.1 | 4.9 | 4.2 | 4.2 | 4.4 | 4.1 | 4.6 | 4.2 |
| | serine | 5.1 | 4.1 | 5.7 | 3.0 | 4.1 | 3.5 | 3.6 | 3.2 | 3.1 | 3.4 | 3.9 | 3.6 | 3.8 | 3.3 | 3.7 | 5.0 | 3.5 | 3.8 |
| | glutamic acid* | 14.5 | 15.2 | 11.2 | 10.4 | 13.5 | 12.6 | 15.5 | 10.8 | 11.6 | 14.7 | 10.9 | 11.2 | 14.1 | 15.8 | 12.6 | 12.2 | 12.7 | 13.0 |
| | proline | 3.5 | 7.3 | 1.0 | 2.5 | 6.0 | 6.4 | 5.0 | 3.5 | 6.8 | 2.8 | 4.7 | 3.3 | 5.3 | 6.1 | 6.9 | 2.7 | t | 2.6 |
| | glycine* | 8.6 | 6.0 | 9.8 | 7.9 | 7.3 | 6.5 | 7.1 | 8.0 | 7.4 | 7.3 | 7.1 | 6.2 | 7.1 | 6.4 | 7.4 | 6.7 | 8.1 | 9.4 |
| | alanine* | 9.0 | 9.5 | 12.4 | 6.5 | 8.2 | 10.0 | 11.0 | 9.7 | 10.1 | 8.4 | 11.2 | 10.8 | 7.8 | 8.5 | 10.0 | 7.6 | 11.1 | 9.3 |
| | cystine | t | t | t | t | t | t | 0.1 | t | t | t | t | 0.1 | t | t | t | 0.1 | t | t |
| | valine* | 6.3 | 11.0 | 12.0 | 11.0 | 7.7 | 8.2 | 7.9 | 8.3 | 6.9 | 10.1 | 9.0 | 6.7 | 7.0 | 8.5 | 9.1 | 10.5 | 10.3 | 9.7 |
| | methionine | 0.9 | 1.0 | t | 3.6 | 2.6 | 2.3 | 1.6 | 2.7 | 1.9 | 2.2 | 3.1 | 2.4 | 3.3 | 1.4 | 0.8 | 1.3 | 2.9 | 1.1 |
| | cystathio-nine | 1.0 | 2.5 | 0.2 | t | 0.5 | 1.1 | 2.1 | 1.9 | 2.4 | 2.3 | 1.6 | 1.2 | 0.9 | 1.4 | t | 1.0 | 2.7 | 1.8 |
| | isoleucine* | 3.3 | 3.6 | 8.2 | 3.7 | 5.9 | 3.8 | 3.1 | 4.2 | 5.1 | 3.2 | 5.6 | 4.3 | 5.7 | 6.6 | 5.0 | 6.9 | 3.9 | 4.8 |

0 058 093

16

TABLE 5 (Continued)

| Item | Example No. | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|---|
| Amino acid component (% by weight) | leucine* | 8.0 | 8.7 | 11.5 | 12.6 | 10.4 | 9.5 | 10.5 | 8.1 | 12.0 | 9.2 | 10.1 | 11.1 | 12.1 | 9.6 | 9.2 | 9.4 | 8.8 | 9.2 |
| | tyrosine | 0.3 | 4.3 | 0.7 | 1.8 | 3.2 | 2.6 | 1.1 | 4.2 | 0.9 | 2.6 | 3.6 | 0.3 | 3.9 | 4.1 | 3.2 | 0.2 | 0.5 | 2.9 |
| | phenylala-nine* | 7.5 | 4.2 | 3.3 | 8.4 | 6.0 | 6.9 | 4.4 | 5.8 | 6.5 | 4.7 | 4.1 | 5.9 | 6.2 | 4.6 | 5.8 | 5.7 | 5.3 | 6.0 |
| | tryptophan | t | t | 0.2 | t | t | 0.1 | t | t | t | 0.1 | t | t | t | t | 0.1 | 0.2 | t | t |
| | ornithine | 0.3 | 0.4 | 1.3 | 0.5 | 1.5 | 1.0 | 1.0 | 0.3 | 0.9 | 1.3 | 1.4 | 1.1 | 0.9 | 1.0 | 1.6 | 0.8 | 1.1 | 1.4 |
| | lysine | 3.5 | 1.2 | 1.2 | 1.8 | 2.9 | 2.8 | 2.2 | 1.6 | 2.9 | 2.4 | 1.9 | 2.9 | 1.8 | 2.0 | 3.1 | 2.6 | 2.3 | 2.8 |
| | histidine | 1.7 | 1.0 | 0.7 | 0.3 | 2.0 | 1.9 | 0.5 | t | 0.9 | 1.2 | 0.4 | 1.7 | t | 1.8 | 0.7 | 1.1 | 1.8 | 0.6 |
| | arginine | 1.8 | 2.0 | 1.1 | 3.5 | 1.7 | 1.9 | 2.6 | 1.9 | 1.9 | 2.0 | 2.7 | 2.3 | 3.4 | 1.6 | 1.8 | 1.7 | 3.1 | 2.9 |
| | (ammonia) | 6.0 | 1.2 | 2.0 | 1.8 | 2.1 | 1.5 | 2.8 | 3.5 | 3.0 | 1.9 | 2.6 | 4.1 | 1.8 | 1.5 | 2.5 | 5.6 | 2.9 | 1.4 |
| | total sum of those with* | 81.0 | 79.1 | 91.6 | 84.2 | 79.5 | 78.4 | 81.1 | 76.9 | 79.4 | 81.2 | 78.0 | 80.7 | 78.7 | 79.1 | 79.3 | 82.8 | 82.7 | 82.5 |

0 058 093

17

TABLE 5 (Continued)

| Item | | Example No. | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|---|---|
| Presence (+) or absence (−) of amino acid at N-end | tyrosine | | + | + | + | + | + | + | + | + | + | + | + | + | + | + | + | + | + | + |
| | leucine | | + | + | + | + | + | + | + | + | + | + | + | + | + | + | + | + | + | + |
| | alanine | | + | + | + | + | + | + | + | + | + | + | + | + | + | + | + | + | + | + |
| | others | | − | − | − | − | − | − | − | − | − | − | − | − | − | − | − | − | − | − |
| Lectin-like activities | Reactivity to monosaccharide glucose | | + | + | + | + | + | + | + | + | + | + | + | + | + | + | + | + | + | + |
| | mannose | | + | + | + | + | + | + | + | + | + | + | + | + | + | + | + | + | + | + + |
| | rhamnose | | + | + | + | + | + | + | + | + | + | + | + | + | + | + | + | + | + | + + |
| | galactose | | − | − | − | − | − | − | − | − | − | − | − | − | − | − | − | − | − | − |
| | Activity according to presence or absence of $Ca^{++}$ ion in presence of $Ca^{++}$ | | + | + | + | + | + | + | + | + | + | + | + | + | + | + | + | + | + | + |
| | in absence of $Ca^{++}$ | | − | − | − | − | − | − | − | − | − | − | − | − | − | − | − | − | − | − |
| | Agglutination of erythrocytes | | + | + | + | + | + | + | + | + | + | + | + | + | + | + | + | + | + | + |

0 058 093

TABLE 5 (Continued)

| Example No. / Item | | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|---|
| Lectin-like activities | Agglutination of tumour cells | + | + | + | + | + | + | + | + | + | + | + | + | + | + | + | + | + | + |
| | Stimulation of blast transformation | + | + | + | + | + | + | + | + | + | + | + | + | + | + | + | + | + | + |
| | Affinity to the surface of lymphocytes | + | + | + | + | + | + | + | + | + | + | + | + | + | + | + | + | + | + |
| | Exudation of macrophage (chernotaxy) | + | + | + | + | + | + | + | + | + | + | + | + | + | + | + | + | + | + |
| Amino acid sequence from C-end of protein moiety | | | | | | | | | | | | | | | | | | | |
| Presence (+) or absence (−) of leucine-phenyl-alanine-valine | | + | + | + | + | + | + | + | + | + | + | + | + | + | + | + | + | + | + |
| Presence (+) or absence (−) of other amino acid sequence | | − | − | − | − | − | − | − | − | − | − | − | − | − | − | − | − | − | − |

0 058 093

19

TABLE 5 (Continued)

| Example No.<br>Item | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| Specific rotatory Power | −1 | −2 | 0 | +1 | −1 | −8 | +5 | −4 | +2 | −3 | +5 | +2 | 0 | −1 | −2 | +1 | −1 | +3 |
| Ultraviolet absorption<br>E at 280 nm | 52 | 55 | 57 | 50 | 65 | 56 | 63 | 57 | 50 | 45 | 42 | 43 | 46 | 46 | 53 | 53 | 42 | 45 |
| E at 260 nm | 67 | 73 | 69 | 71 | 77 | 67 | 75 | 68 | 56 | 48 | 44 | 47 | 48 | 56 | 69 | 58 | 53 | 55 |
| E280 E260 | 0.77 | 0.76 | 0.82 | 0.70 | 0.85 | 0.83 | 0.84 | 0.84 | 0.90 | 0.93 | 0.94 | 0.91 | 0.95 | 0.82 | 0.77 | 0.91 | 0.79 | 0.81 |
| Content of uracil (% wt.) as base of nucleic acid | 0.27 | 0.45 | 0.30 | 0.40 | 0.36 | 0.24 | 0.32 | 0.31 | 0.19 | 0.16 | 0.04 | 0.15 | 0.02 | 0.21 | 0.41 | 0.19 | 0.23 | 0.22 |
| Content of organic phosphorus (% wt. as $P_2O_5$) | 0.09 | 0.11 | 0.07 | 0.09 | 0.10 | 0.06 | 0.07 | 0.10 | 0.05 | 0.02 | 0.01 | 0.03 | 0.04 | 0.02 | 0.12 | 0.07 | 0.04 | 0.05 |

0 058 093

TABLE 5 (Continued)

| Example No. / Item | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| **Inhibition extent to Sarcoma-180 on mouse by i.p.[1] administration** | | | | | | | | | | | | | | | | | | |
| % at 1 mg/kg/time | 100 | 100 | 100 | 100 | 99 | 100 | 99 | 100 | 100 | 100 | 100 | 99 | 100 | 100 | 100 | 98 | 99 | 99 |
| % at 10 mg/kg/time | 100 | 100 | 100 | 100 | 100 | 100 | 100 | 99 | 100 | 100 | 100 | 100 | 100 | 99 | 100 | 99 | 98 | 99 |
| % at 100 mg/kg/time | 100 | 100 | 100 | 100 | 100 | 99 | 100 | 100 | 100 | 100 | 100 | 100 | 100 | 100 | 100 | 99 | 99 | 99 |
| **by p.o.[2] administration** | | | | | | | | | | | | | | | | | | |
| % at 50 mg/kg/time | 88 | 85 | 83 | 82 | 85 | 81 | 85 | 85 | 87 | 83 | 80 | 83 | 86 | 81 | 81 | 83 | 80 | 80 |
| % at 100 mg/kg/time | 90 | 86 | 86 | 83 | 86 | 84 | 85 | 87 | 88 | 85 | 82 | 84 | 88 | 82 | 81 | 84 | 82 | 82 |
| % at 250 mg/kg/time | 90 | 89 | 90 | 86 | 87 | 87 | 88 | 89 | 90 | 89 | 82 | 86 | 90 | 85 | 85 | 87 | 83 | 85 |
| **Inhibition extent to Ehrlich ascites tumour on mouse by i.p. adminstr.** | | | | | | | | | | | | | | | | | | |
| % at 50 mg/kg/time | 100 | 100 | 100 | 100 | 100 | 100 | 100 | 100 | 100 | 100 | 100 | 100 | 100 | 100 | 100 | 100 | 100 | 100 |
| **Survival rate of mouse transplanted with Ehrlich ascites tumour cells for 20 days** | 8/10 | 8/10 | 8/10 | 8/10 | 7/10 | 8/10 | 8/10 | 8/10 | 8/10 | 8/10 | 8/10 | 8/10 | 8/10 | 8/10 | 7/10 | 8/10 | 8/10 | 8/10 |

Notes: 1) intraperitoneous injection.
2) oral administration.

In addition, each of the substances obtained in Examples 1 to 18 showed the following colour reactions of saccharides and of proteins.

| (1) Colour reaction of saccharides | Colour |
| --- | --- |
| $\alpha$-naphthol—sulphuric acid reaction | purple |
| indole—sulphuric acid reaction | brown |
| anthrone—sulphuric acid reaction | greenish blue |
| phenol—sulphuric acid reaction | brown |
| tryptophan—sulphuric acid reaction | purplish brown |
| thioglycolic acid—sulphuric acid reaction | greenish brown |
| orcinol—hydrochloric acid reaction | greenish brown |

| (2) Colour reaction of proteins | Colour |
| --- | --- |
| Lowry-Folin reaction | blue |
| Ninhydrin reaction on a hydrolyzate of the present substance obtained by the reaction of hydrochloric acid | purplish brown |

According to infrared absorption spectroscopy, each substance showed absorption maxima in ranges 3600 to 3200 cm$^{-1}$ and 1200 to 1000 cm$^{-1}$, and at 1600 and 1530 cm$^{-1}$.

## Claims

1. A glycoprotein having a molecular weight of from 5,000 to 300,000 as determined by the ultracentrifugation method; the ratio of the weight of its protein moiety, as determined by Lowry-Folin's method, to the weight of its saccharide moiety, as determined by the phenolsulfuric acid method, being from 50:50 to 80:20; the saccharide moiety containing fucose, ribose, arabinose, xylose, mannose, galactose, glucose and glucosamine, and the total weight of said xylose, said mannose and said glucose is more than 85% by weight of the total weight of said saccharides; the protein moiety containing aspartic acid, threonine, serine, glutamic acid, proline, glycine, alanine, cysteine, valine, methionine, cystathionine, isoleucine, leucine, tyrosine, phenylalanine, tryptophan, ornithine, lysine, histidine, and arginine, and the total weight of said aspartic acid, said threonine, said serine, said glutamic acid, said glycine, said alanine, said phenylalanine, said valine, said leucine, and said isoleucine is more than 75% by weight of the total weight of all said amino acids; the amino acid at its N-end being tyrosine, leucine or alanine; the amino acid sequence at its C-end being leucine to phenylalanine to valine, the terminal amino acid being leucine; its elementary composition being from 35.2 to 49.3% of C, from 4.8 to 8.0% of H, from 4.3 to 12.3% of N, from a trace amount to 2.5% of S, from a trace amount to 1.2% of P and the balance being O; its isoelectric point being from pH 2.5 to pH 5.0; and having nucleic acid component.

2. A glycoprotein according to claim 1, wherein the protein moiety consists of 10 to 20% by weight of aspartic acid, 4 to 6% by weight of threonine, 3 to 6% by weight of serine, 10 to 16% by weight of glutamic acid, a trace amount to 8% by weight of proline, 6 to 10% by weight of glycine, 6 to 13% by weight of alanine, a trace amount of cysteine, 5 to 12% by weight of valine, a trace amount to 4% by weight of methionine, a trace amount to 3% by weight of cystathionine, 3 to 8% by weight of isoleucine, 8 to 13% by weight of leucine, a trace amount to 5% by weight of tyrosine, 3 to 9% by weight of phenylalanine, a trace amount to 1% by weight of tryptophan, a trace amount to 2% by weight of ornithine, 1 to 4% by weight of lysine, a trace amount to 2% by weight of histidine and 1 to 4% by weight of arginine.

3. A glycoprotein according to claim 1 or 2 wherein the saccharide moiety consists of 0.5 to 4.0% by weight of fucose, a trace amount to 2.0% by weight of ribose, 0.3 to 6.0% by weight of arabinose, 2.0 to 40.0% by weight of xylose 5.0 to 20.0% by weight of mannose, 0.5 to 8.0% by weight of galactose, 30.0 to 80.0% by weight of glucose and a trace amount to 3.0% by weight of glucosamine.

4. A glycoprotein according to any one of the proceeding claims, wherein the nucleic acid component comprises uracil as a nucleic acid base in an amount of 0.01 to 0.50% by weight of the glycoprotein.

5. A glycoprotein according to any one of the proceeding claims for use in the treatment of a tumour.

6. A process for producing a glycoprotein according to any one of claims 1 to 4 by extracting fruit bodies or mycelia of a basidiomycetous fungus belonging to the genus *Coriolus* with hot water or an aqueous 0.01 to 2.0N alkali solution subjecting the resultant extract to dialysis and/or ultra-filtration thereby removing a low molecular weight substance(s) having a molecular weight below 5,000; characterised in that the process further comprises collecting the fraction precipitating at a pH in the range of from 2.5 to 5.0 at the isoelectric point of the glycoprotein.

7. A process according to claim 6, wherein the basidiomycetous fungus is selected from *Coriolus versicolor* (Fr.) Quél., *Coriolus hirsutus* (Fr.) Quél., *Coriolus pergamenus* (Fr.) Pat., *Coriolus consors* (Berk.) Imaz., *Coriolus pubescens* (Fr.) Quél., *Coriolus biformis* (Klotz.) Pat. and *Coriolus conchifer* (Schw.) Pat.

8. A process according to claim 6 or 7 wherein the step of extracting is carried out at a temperature of from 80 to 100°C for from 1 to 8 hours.

9. A process according to any one of claims 6 to 8, wherein the glycoprotein is collected by the method of fractional precipitation at isoelectric points while adjusting the pH of the glycoprotein solution to from 2.5 to 5.0.

10. A process according to claim 9, wherein the method of fractional precipitation at the isoelectric point is carried out on a solution of ion strength of from 0.1 to 3.1 $\mu$ at a temperature of from 5 to 25°C and for more than 0.5 hours.

11. A process according to any one of claims 6 to 10 wherein the fraction collected by precipitation is neutralized and subjected to dialysis or ultrafiltration.

12. A pharmaceutical composition which comprises a glycoprotein as claimed in any one of claims 1 to 5, or which has been produced by a process as claimed in any one of claims 6 to 11, as active ingredient, together with a pharmaceutically acceptable carrier or diluent.

13. A pharmaceutical composition according to claim 12 which is in unit dosage form.

**Revendications**

1. Une glycoprotéine ayant un poids moléculaire, déterminé selon la méthode d'ultracentrifugation, de 5 000 à 300 000, le rapport du poids de sa portion protéine, comme déterminé selon la méthode de Lowry-Folin, au poids de sa portion saccharide, comme déterminé selon la méthode à l'acide phénolsulfurique, étant de 50/50 à 80/20; la portion saccharide contenant du fucose, du ribose, de l'arabinose, du xylose, du mannose, du galactose, du glucose et de la glucosamine, et le poids total dudit xylose, dudit mannose et dudit glucose étant supérieur à 85% du poids total desdits saccharides; la portion protéine contenant de l'acide aspartique, de la thréonine, de la sérine, de l'acide glutamique, de la proline, de la glycine, de l'alanine, de la cystéine, de la valine, de la méthionine, de la cystathionine, de l'isoleucine, de la leucine, de la tyrosine, de la phénylalanine, du tryptophane, de l'ornithine, de la lysine, de l'histidine et de l'arginine, et le poids total dudit acide aspartique, de ladite thréonine, de ladite sérine, dudit acide glutamique, de ladite glycine, de ladite alanine, de ladite phénylalanine, de ladite valine, de ladite leucine et de ladite isoleucine étant supérieur à 75% du poids total de tous lesdits amino-acides; l'amino-acide de l'extrémité N-terminale étant la tyrosine, la leucine ou l'alanine; la séquence des amino-acides de l'extrémité C-terminale étant le leucine à la phénylalanine à la valine, l'amino-acide terminal étant la leucine, sa composition élémentaire étant de 35,2 à 49,3% de C, de 4,8 à 8,0% de H, de 4,3 à 12,3% de N, de traces à 2,5% de S, de traces à 1,2% de P, le reste étant de 1,0; son point isoélectrique étant de pH 2,5 à pH 5,0; et ayant un composant acide nucléique.

2. Une glycoprotéine selon la revendication 1, dans laquelle la portion protéine est constituée de 10 à 20% en poids d'acide aspartique, 4 à 6% en poids de thréonine, 3 à 6% en poids de sérine, 10 à 16% en poids d'acide glutamique, de traces à 8% en poids de proline, de 6 à 10% en poids de glycine, de 6 à 13% en poids d'alanine, de traces de cystéine, de 5 à 12% en poids de valine, de traces à 4% en poids de méthionine, de traces à 3% en poids de cystathionine, de 3 à 8% en poids d'isoleucine, de 8 à 13% en poids de leucine, de traces à 5% en poids de tyrosine, de 3 à 9% en poids de phénylalanine, de traces à 1% en poids de tryptophane de traces à 2% en poids d'ornithine, de 1 à 4% en poids de lysine, de traces à 2% en poids d'histidine et de 1 à 4% en poids d'arginine.

3. Une glycoprotéine selon la revendication 1 ou 2, dans laquelle la portion saccharide est constituée de 0,5 à 4,0% en poids de fucose, de traces à 2,0% en poids de ribose, de 0,3 à 6,0% en poids d'arabinose, de 2,0 à 40,0% en poids de xylose, de 5,0 à 20,0% en poids de mannose, de 0,5 à 8,0% en poids de galactose, de 30,0 à 80,0% en poids de glucose et de traces à 3,0% en poids de glucosamine.

4. Une glycoprotéine selon l'une quelconque des revendications précédentes, dans laquelle le composant acide nucléique comprend de l'uracile comme base d'acide nucléique en une quantité de 0,01 à 0,50% en poids de la glycoprotéine.

5. Une glycoprotéine selon l'une quelconque des revendications précédentes, pour l'emploi dans le traitement d'une tumeur.

6. Un procédé pour la production d'une glycoprotéine, selon l'une quelconque des revendications 1 à 4, par extraction des appareils sporifères ou des mycéliums d'un champion basidiomycète appartenant au genre *Coriolus* avec de l'eau chaude ou avec une solution aqueuse d'alcali 0,01 à 2,0 N, en soumettant l'extrait obtenu à une dialyse et/ou une ultra-filtration pour éliminer une (des) substance(s) de bas poids moléculaire ayant un poids moléculaire inférieur à 5 000; caractérisé en ce que le procédé comprend de plus la récolte de la fraction précipitant à un pH dans la gamme de 2,5 à 5,0 au point isoélectrique de la glycoprotéine.

7. Un procédé selon la revendication 6, dans lequel le champion basidiomycète est choisi parmi *Corilus versicolor* (Fr.) Quel., *Coriolus hisutus* (Fr.) Quel., *Coriolus pergamenus* (Fr.) Pat., *Coriolus consors* (Berk.) Imaz., *Coriolus pubescens* (Fr.) Quel., *Coréolus biformis* (Klotz.) Pat. et *Coriolus conchifer* (Schw.) Pat.

8. Un procédé selon la revendication 6 ou 7, dans lequel le stade d'extraction est effectué à une température de 80 à 100°C pendant 1 à 8 heures.

9. Un procédé selon l'une quelconque des revendications 6 à 8, dans lequel la glycoprotéine est recueillie selon le procédé de précipitation fractionnée aux points isoélectriques avec ajustement du pH de la solution de glycoprotéine de 2,5 à 5,0.

10. Un procédé selon la revendication 9, dans lequel le procédé de précipitation fractionnée au point isoélectrique est effectué sur une solution ayant une force ionique de 0,1 à 3,1 $\mu$ à une température de 5 à 25°C et pendant plus de 0,5 heure.

11. Un procédé selon l'une quelconque des revendications 6 à 10, dans lequel la fraction recueillie par précipitation est neutralisée et soumise à une dialyse ou une ultrafiltration.

12. Une composition pharmaceutique qui comprend une glycoprotéine comme revendiquée dans l'une quelconque des revendications 1 à 5 ou que l'on a produite selon un procédé comme revendiqué dans l'une quelconque des revendications 6 à 11, comme ingrédient actif, avec un support ou diluant acceptable en pharmacie.

13. Une composition pharmaceutique selon la revendication 12, qui est sous forme d'une dose d'administration unitaire.

## Patentansprüche

1. Glycoprotein mit einem Molekulargewicht von 5000 bis 300 000 gemäß Bestimmung nach der Ultrazentrifugen-Methode, wobei das Verhältnis des Gewichtes seines Proteinteils, wie es durch das Lowry-Folin-Verfahren bestimmt wurde, zum Gewicht seines Saccharidteils, wie es nach der Phenolschwefelsäure-Methode bestimmt wurde, von 50:50 bis 80:20 beträgt, wobei der Saccharidteil Fucose, Ribose, Arabinose, Xylose, Mannose, Galactose, Glucose und Glucosamin enthält, und das Gesamtgewicht der Xylose, der Mannose und der Glucose mehr als 85 Gew.-% des Gesamtgewichts der Saccharide beträgt, wobei der Proteinteil, der Asparaginsäure, Threonin, Serin, Glutaminsäure, Prolin, Glycin, Alanin, Cystein, Valin, Methionin, Cystathionin, Isoleucin, Leucin, Tyrosin, Phenylalanin, Tryptophan, Ornithin, Lysin, Histidin und Arginin enthält, und das Gesamtgewicht der Asparaginsäure, des Threonins, des Serins, der Glutaminsäure, des Glycins, des Alanins, des Phenylalanins, des Valins, des Leucins und des Isoleucins mehr als 75 Gew.-% des Gesamtgewichtes aller genannten Aminosäuren beträgt, wobei die Aminosäure an ihrem N-Ende Tyrosin, Leucin oder Alanin ist, wobei die Aminosäurensequenz an ihrem C-Ende Leucin zu Phenylalanin zu Valin ist, wobei die endständige Aminosäure Leucin ist; wobei seine Elementar-Zusammensetzung 35,2 bis 49,3% C, 4,8 bis 8,0% H, 4,3 bis 12,3% N, Spurenmengen bis 2,5% S, Spurenmengen bis 1,2% P beträgt und der Rest O ist; sein isoelektrischer Punkt bei pH 2,5 bis pH 5,0 liegt, und es eine Nukleinsäure-Komponente enthält.

2. Glycoprotein nach Anspruch 1, worin der Proteinteil aus 10 bis 20 Gew.-% Asparaginsäure, 4 bis 6 Gew.-% Threonin, 3 bis 6 Gew.-% Serin, 10 bis 16 Gew.-% Glutaminsäure, eine Spurenmenge bis 8 Gew.-% Prolin, 6 bis 10 Gew.-% Glycin, 6 bis 13 Gew.-% Alanin, eine Spurenmenge an Cystein, 5 bis 12 Gew.-% Valin, eine Spurenmenge bis 4 Gew.-% Methionin, eine Spurenmenge bis 3 Gew.-% Cystathionin, 3 bis 8 Gew.-% Isoleucin, 8 bis 13 Gew.-% Leucin, eine Spurenmenge bis 5 Gew.-% Tyrosin, 3 bis 9 Gew.-% Phenylalanin, eine Spurenmenge bis 1 Gew.-% Tryptophan, eine Spurenmenge bis 2 Gew.-% Ornithin, 1 bis 4 Gew.-% Lysin, eine Spurenmenge bis 2 Gew.-% Histidin und 1 bis 4 Gew.-% Arginin besteht.

3. Glycoprotein nach Anspruch 1 oder 2, worin der Saccharidteil aus 0,5 bis 4,0 Gew.-% Fucose, einer Spurenmenge bis 2,0 Gew.-% Ribose, 0,3 bis 6 Gew.-% Arabinose, 2,0 bis 40,0 Gew.-% Xylose, 5,0 bis 20,0 Gew.-% Mannose, 0,5 bis 8,0 Gew.,-% Galactose, 30,0 bis 80,0 Gew.-% Glucose und einer Spurenmenge bis 3,0 Gew.-% Glucosamin besteht.

4. Glycoprotein nach einem der voranstehenden Ansprüche, worin die Nukleinsäure-Komponente Uracil als eine Nukleinsäure-Base in einer Menge von 0,01 bis 0,50 Gew.-% des Glycoproteins enthält.

5. Glycoprotein nach einem der voranstehenden Ansprüche zur Verwendung bei der Behandlung eines Tumors.

6. Verfahren zur Herstellung eines Glycoproteins nach einem der Ansprüche 1 bis 4 durch Extraktion der Fruchtkörper oder Mycelia eines Pilzes Basidiomycetous, der zur Gattung Coriolus gehört, mit heißem Wasser oder einer wässrigen 0,01 bis 2,0N Alkalilösung, Unterwerfung des erhaltenen Extrakts unter eine Dialyse und/oder Ultra-Filtration, wodurch eine Substanz(en) niederen Molekulargewichts mit einem Molekulargewicht unterhalb von 5000 entfernt wird, dadurch gekennzeichnet, daß das Verfahren ferner das Sammeln der Fraktion umfaßt, die bei einem pH-Wert im Bereich von 2,5 bis 5,0 am isoelektrischen Punkt des Glycoproteins ausfällt.

7. Verfahren nach Anspruch 6, worin der Pilz Basidiomycetous ausgewählt ist unter Coriolus versicolor (Fr.) Quel., Coriolus hirsutus (Fr. Quel., Coriolus pergamenus (Fr.) Pat., Coriolus consors (Berk.) Imaz., Coriolus pubescens (Fr.) Quel., Coriolus biformis (Klotz.) Pat. und Coriolus conchifer (Schw.) Pat.

8. Verfahren nach Anspruch 6 oder Anspruch 7, worin die Stufe der Extraktion bei einer Temperatur von 80 bis 100°C für 1 bis 8 Stunden durchgeführt wird.

9. Verfahren nach einem der Ansprüche 6 bis 8, worin das Glycoprotein nach dem Verfahren der fraktionierten Ausfällung an isoelektrischen Punkten gesammelt wird, wobei der pH-Wert der Glycoprotein-Lösung auf 2,5 bis 5,0 eingestellt wird.

10. Verfahren nach Anspruch 9, worin das Verfahren der fraktionierten Ausfällung am isoelektrischen Punkt an einer Lösung der Ionenstärke von 0,1 bis 3,1 $\mu$ bei einer Temperatur von 5 bis 25°C und für mehr als 0,5 Stunden durchgeführt wird.

11. Verfahren nach einem der Ansprüche 6 bis 10, worin die Fraktion, die durch Ausfällen gesammelt wurde, neutralisiert wird und einer Dialyse oder Ultra-Filtration unterworfen wird.

12. Pharmazeutische Zusammensetzung, die ein Glycoprotein nach einem der Ansprüche 1 bis 5 oder ein Glycoprotein, welches nach einem Verfahren gemäß einem der Ansprüche 6 bis 11 hergestellt wurde, als aktiven Bestandteil zusammen mit einem pharmazeutisch verträglichen Träger oder Verdünnungsmittel enthält.

13. Pharmazeutische Zusammensetzung nach Anspruch 12, die in einer Einheitsdosisform vorliegt.

# F I G. 1

# FIG. 2

# FIG. 3

# FIG. 4

# FIG. 5

# FIG. 6

WAVELENGTH ( μm )

# FIG. 7

WAVELENGTH ( μm )

# FIG. 8

WAVELENGTH ( μm )

# FIG. 9

WAVELENGTH ( μm )

# FIG. 10

# FIG. 11

# F I G . 12

# F I G . 13

# FIG. 14

# FIG. 15

# FIG.16

WAVELENGTH (μm)

# FIG.17

WAVELENGTH (μm)

# FIG. 18

WAVELENGTH (µm)

# FIG. 19

WAVELENGTH (µm)

FIG. 20